Europäisches Patentamt

**European Patent Office**

**Office européen des brevets**

(19)

(11) Numéro de publication: **0 068 929**
A2

# DEMANDE DE BREVET EUROPEEN

(12)

(21) Numéro de dépôt: **82400987.2**

(51) Int. Cl.³: **A 61 B 6/00**

(22) Date de dépôt: **27.05.82**

(30) Priorité: **10.06.81 BE 2059205**

(43) Date de publication de la demande: **05.01.83**
**Bulletin 83/1**

(84) Etats contractants désignés: **DE FR IT NL**

(71) Demandeur: **THOMSON-CSF, 173, Boulevard Haussmann, F-75379 Paris Cedex 08 (FR)**

(72) Inventeur: **Munch, Joseph, THOMSON-CSF SCPI 173, bld Haussmann, F-75379 Paris Cedex 08 (FR)**

(74) Mandataire: **Barbin le Bourhis, Joel et al, THOMSON-CSF SCPI 173, boulevard Haussmann, F-75379 Paris Cedex 08 (FR)**

(54) **Dispositif à utiliser pour maintenir en équilibre un élément suspendu et déplaçable en hauteur, tel q'un téléscope associé à un porte-tube Rontgen.**

(57) Le dispositif susdit comporte en ordre principal un «ressort gazeux» (12) pour maintenir en équilibre l'élément suspendu (1), un servomoteur (6) pour neutraliser la force de frottement du «ressort gazeux» (12), un moyen de compensation (4) pour compenser dans toutes les positions du «ressort gazeux» (12) les forces variables de celui-ci et pouvoir en conséquence maintenir constamment en équilibre l'élément (1) suspendu, ledit dispositif comprenant en plus un mécanisme de transmission (3 - 5 - 7 - 8 - 9 - 10) placé entre l'élément suspendu (1), le «ressort gazeux» (12), le moyen de compensation (4) et le servomoteur (6)

## DISPOSITIF A UTILISER POUR MAINTENIR EN EQUILIBRE
## UN ELEMENT SUSPENDU ET DEPLACABLE EN HAUTEUR,
## TEL QU'UN TELESCOPE ASSOCIE A UN PORTE-TUBE RONTGEN

L'invention a pour objet un dispositif à utiliser pour maintenir en équilibre un élément suspendu dans un appareil Röntgen et déplaçable en hauteur à la main, à savoir un appareil tel qu'un télescope avec porte-tube Röntgen, un amplificateur vidéo, un récepteur d'images et appareils analogues.

On connaît des dispositifs dans lesquels il est fait usage d'un mécanisme à câble et à ressorts en spirale ou à ressorts de traction ou d'un moteur, placé entre l'élément déplaçable en hauteur et la partie fixe de l'appareil, cela pour maintenir en équilibre l'élément susnommé.

Attendu que, dans de nombreux cas, l'élément suspendu est relativement lourd, on est forcé d'utiliser des mécanismes lourds et volumineux pour pouvoir maintenir le tout en équilibre. Il s'ensuit que le prix de revient de ces dispositifs est assez élevé. Ceux-ci ne sont en outre pas toujours efficaces et le réglage manuel des éléments déplaçables en hauteur ne peut pas toujours d'effectuer d'une manière précise.

Suivant la caractéristique principale de l'invention et pour obvier à ces inconvénients, on a réalisé un dispositif qui, en ordre principal comporte un "ressort gazeux" ou un cylindre rempli d'un gaz sous pression et fonctionnant dans deux directions, cylindre complété par un piston et une bielle de piston, cela pour maintenir en équilibre l'élément suspendu, un servomoteur nécessaire pour neutraliser la force de frottement du piston dans le "ressort gazeux" pendant le déplacement manuel en hauteur de l'élément suspendu, un moyen de compensation nécessaire pour compenser dans toutes les positions du "ressort gazeux" les forces variables de celui-ci et pouvoir en conséquence maintenir constamment en équilibre dans toutes ses positions en hauteur, l'élément suspendu, le dispositif susdit comportant en plus un mécanisme de transmission placé entre l'élément suspendu, le "ressort gazeux", le moyen de compensation et le servomoteur.

Ce dispositif a donc l'avantage d'être très compact, de n'être pas lours et d'être bon marché dans son exécution.

Le texte ci-après fournit à titre d'exemple une description plus détaillée d'une forme de réalisation du dispositif susdit conforme à l'invention, une telle forme n'étant qu'une forme possible de celle-ci mais en aucune façon limitée à celle-ci. A la susdite description, il est joint des dessins dans lesquels :

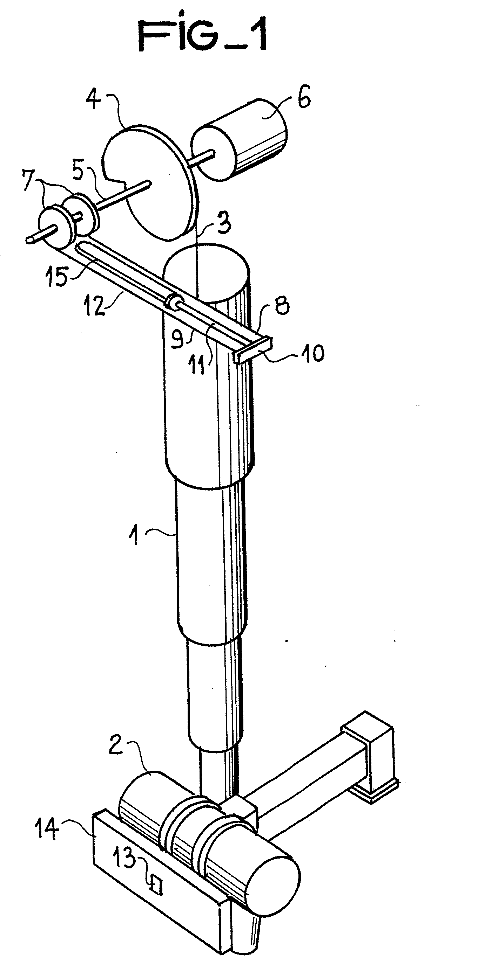

- la figure 1 représente schématiquement, en perspective, le dispositif susdit ;

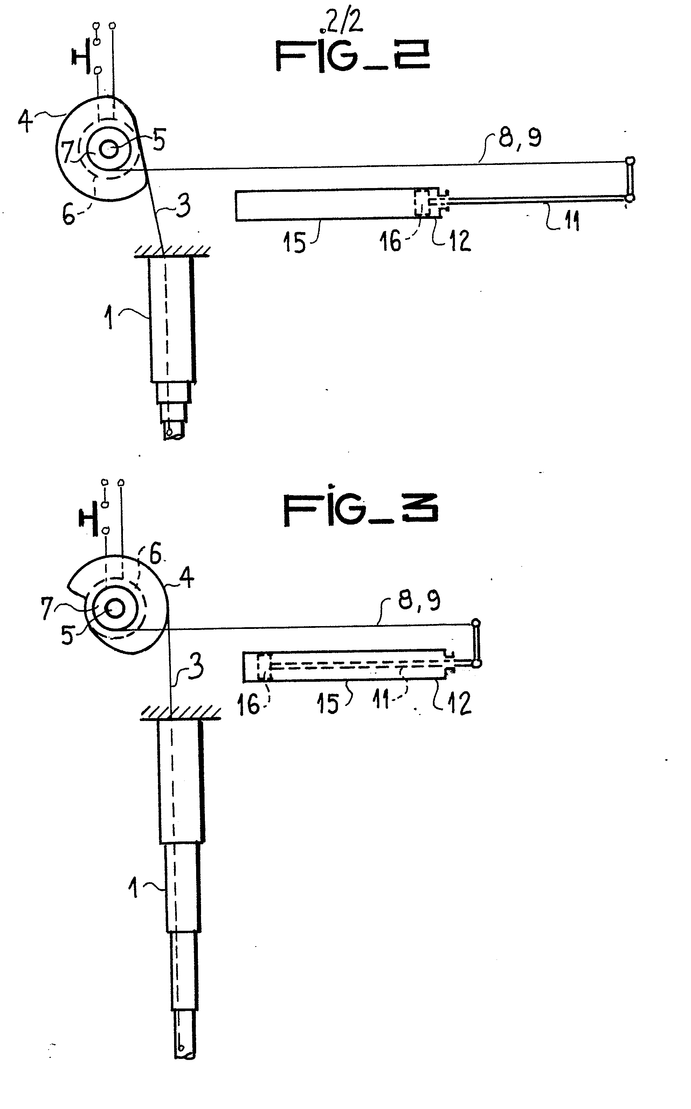

- la figure 2, une vue schématique du dispositif susdit, à savoir une vue, dans laquelle le télescope suspendu est représenté en position "rentrée" ;

- la figure 3 est une vue schématique du dispositif susdit, à savoir une vue dans laquelle le télescope suspendu est représenté en position "retirée"

Dans ces figures, on remarque que la partie inférieure rentrable et extensible du télescope 1 sur laquelle il est monté dans le présent cas un porte-tube Röntgen 2, est suspendue à un câble de traction 3 fixé à un enrouleur de câble 4 de forme excentrique et pouvant être enroulé sur celui-ci. Cet enrouleur de câble 4 de forme excentrique est monté sur l'axe 5 d'un servomoteur 6, de manière à être solidaire de cet axe. Sur l'axe 5, il est également monté deux enrouleurs 7, de manière à être également solidaires de cet axe. A chacun de ces enrouleurs 7, il est fixé un câble respectivement 8 - 9, ces câbles étant d'autre part chacun attachés à une traverse 10, montée sur la bielle de piston 11, d'un ressort gazeux 12 qui maintient le tout en équilibre. Il est placé sur le panneau 14 de l'appareil, un bouton-poussoir 13 qui sert à faire démarrer le servomoteur 6 et à le mettre à l'arrêt. Les dimensions et la forme de l'enrouleur de câble 4 de forme excentrique sur lequel le câble de traction 3 est enroulé sont choisies de telle manière que dans une position quelconque, occupée par la bielle 11 de piston par rapport au cylindre 15 du ressort gazeux 12 et dans laquelle la force exercée par le ressort gazeux varie, la force de traction ou la force de compression à exercer sur le télescope 1 à déplacer en hauteur et sur le câble à traction 3, soient cependant toujours constantes, cela de manière à atteindre dans toutes les positions du télescopes 1, un équilibre parfait.

Pour pouvoir manier facilement le télescope 1 sans devoir dépenser une force manuelle exagérée et pour régler d'une manière précise et à une hauteur déterminée le porte-tube Röntgen 2 y suspendu et la lampe Röntgen, il est prévu le servomoteur 6 pour veiller à ce que la résistance de frottement du ressort gazeux 12 soit vaincue à chaque déplacement du télescope 1.

Pour le réglage en hauteur du porte-tube Röntgen 2, on enfonce le bouton-poussoir 13 monté sur le panneau 14 et ainsi le moteur 6 démarre et le télescope 1 est rentré ou retiré à la main. La résistance de frottement du ressort gazeux 12 est immédiatement vaincue par le servomoteur 6 dont la force à exercer par lui est juste suffisante pour neutraliser cette résistance. Le télescope 1 peut être alors placé sans peine dans une position déterminée tandis que les câbles 8 - 9 sont enroulés sur les enrouleurs 7 ou dévidés de ceux-ci et que le câble de traction 3 est enroulé sur l'enrouleur de câble 4 ou dévié de celui-ci par la force de traction du ressort gazeux 12 qui maintient le tout en équilibre. L'enrouleur de câble 4 de forme excentrique veille à ce que la force de traction exercée sur le câble de traction 3 soit toujours constante, cela de telle manière que le télescope 1 puisse être réglé en hauteur dans toutes positions en hauteur au moyen d'une même force manuelle.

Il va sans dire que la forme, les dimensions et le montage mutuel de ces éléments ci-avant décrits peuvent différer et que des pièces déterminées pourraient être remplacées par d'autres équivalentes, cela à condition de ne pas sortir du cadre de l'invention.

- 4 -                    0068929

<u>R E V E N D I C A T I O N S</u>

1. Dispositif à utiliser pour maintenir en équilibre un élément suspendu et déplaçable en hauteur, par exemple un élément tel qu'un télescope (1) avec porte-tube Röntgen (2), caractérisé par le fait qu'en ordre principal, le dispositif susdit comporte un "ressort gazeux" (12) pour maintenir en équilibre l'élément suspendu (1), un servomoteur (6) pour neutraliser la force de frottement du piston (16) dans le "ressort gazeux" (12) pendant le déplacement manuel en hauteur de l'élément suspendu (1), un moyen de compensation (4) pour compenser dans toutes les positions du "ressort gazeux" (12) les forces variables de celui-ci et pour maintenir constamment en équilibre dans toutes ses positions en hauteur l'élément suspendu, et un mécanisme de transmission (3 - 5 - 7 - 8 - 9 - 10) placé entre l'élément suspendu (1), le "ressort gazeux" (12), le moyen de compensation (4) et le servomoteur (6).

2. Dispositif conforme à la revendication 1, caractérisé par le fait que le mécanisme de transmission comporte un axe de rotation (5), au moins un enrouleur (7) fixé sur cet axe de rotation (5), au moins un câble (8 - 9) prévu entre l'enrouleur (7) et la bielle de piston (11) du "ressort gazeux" (12), tandis que sont couplés à l'axe de rotation (5) le moyen de compensation (4) et le servomoteur (6).

3. Dispositif conforme à la revendication 2, caractérisé par le fait que le moyen de compensation comporte un enrouleur de câble (4) de forme excentrique fixé sur l'axe de rotation (5), auquel enrouleur est fixé un câble de traction (3) auquel est suspendu l'élément (1) réglable en hauteur.

4. Dispositif conforme à la revendication 2, caractérisé par le fait que sur l'axe de rotation (5) sont fixés deux enrouleurs (7), tandis qu'à chaque enrouleur (7) il est fixé un câble (8 -9) fixé d'autre part à une traverse (10) montée sur la bielle de piston (11) du "ressort gazeux" (12).

FIG_1

FIG_2

FIG_3